(19) Europäisches Patentamt
European Patent Office
Office européen des brevets

(11) Numéro de publication: **0 063 064**
B1

(12) # FASCICULE DE BREVET EUROPÉEN

(45) Date de publication du fascicule du brevet: **02.07.86**

(51) Int. Cl.⁴: **G 01 N 33/531**, G 01 N 33/548

(21) Numéro de dépôt: **82400519.3**

(22) Date de dépôt: **23.03.82**

(54) **Réactif pour le dosage radio-ou enzymo immunologique des IgE.**

(30) Priorité: **24.03.81 FR 8105877**

(43) Date de publication de la demande:
**20.10.82 Bulletin 82/42**

(45) Mention de la délivrance du brevet:
**02.07.86 Bulletin 86/27**

(84) Etats contractants désignés:
**AT BE CH DE GB IT LI LU NL SE**

(56) Documents cité:
**EP-A-0 011 030**
**FR-A-1 588 874**
**FR-A-2 331 567**
**FR-A-2 382 458**
**FR-A-2 384 262**
**FR-A-2 403 098**
**FR-A-2 410 012**
**FR-A-2 445 966**
**US-A-4 256 833**

**CHEMICAL ABSTRACTS, vol. 90, no. 15, 09 avril 1979, abrégé 119481f, page 467, COLUMBUS OHIO (US), B. FERRUA et al.: "Coupling of gamma-globulin to microcrystalline cellulose by periodate oxidation"**
**CHEMICAL ABSTRACTS, vol. 85, no. 17, 25 octobre 1976, abrégé 118992h, page 247, COLUMBUS OHIO (US), M.B. WILSON et al.: "The covalent coupling**

(73) Titulaire: **LABORATOIRE DES STALLERGENES, 7 allée des Platanes, F-94250 Fresnes (FR)**

(72) Inventeur: **Guerin, Bernard, 160 Quai de Polangis, F-94340 Joinville le Pont (FR)**
Inventeur: **Lelievre, Danièle, 160 Quai de Polangis, F-94340 Joinville le Pont (FR)**

(74) Mandataire: **Bernasconi, Jean, CABINET LEMOINE ET BERNASCONI 13, BLd. des Batignolles, F-75008 Paris (FR)**

(56) Documents cité: (suite)
**of proteins to periodate-oxidized Sephadex: a new approach to immunoadsorbent preparation"**
**C.J. SANDERSON et al., Immunology, 1971, 20, 1061-1065**

EP 0 063 064 B1

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

LIBER, STOCKHOLM 1986

## Description

L'invention a pour objet un réactif pour le dosage radio- ou enzymo- immunologique des IgE.

Les déterminations immunologiques des anticorps et des antigènes (immunoglobulines par exemple) présents dans le sérum sanguin peuvent être effectuées principalement selon deux methodes:

Selon une première méthode, pour doser les anticorps spécifiques d'un antigène donné, et en particulier les réagines specifiques d'un allergène, on utilise un support insoluble dans l'eau, ou immunoadsorbant, sur lequel a été fixé l'antigène contre lequel est dirigé spécifiquement l'anticorps à doser. Dans une première étape, ce support est mis en contact avec le sérum à doser: les anticorps specifiques de l'antigène fixé sur le support se combinent avec celuici. Après lavage, le support est, dans une deuxième étape, mis en contact avec un anticorps dirigé spécifiquement contre les anticorps ou immunoglobulines à doser, et qui a été marqué, soit par un élément radioactif, soit par un enzyme. Après lavage, on mesure dans le premier cas la radioactivité (test radio-immunologique), dans le second cas, l'activité enzymatique, fixée sur le support par colorimétrie (test enzymo-immunologique).

Selon une deuxième méthode, dite "sandwich", pour doser en particulier certaines immunoglobulines du sérum, qui jouent alors le rôle d'antigènes, on utilise également un support insoluble, mais sur lequel a été fixé l'anticorps dirigé contre les immunoglobulines à doser (par exemple, anti-IgE totales ou anti-réagines, ou anti-IgG). Ce support est ensuite mis en contact avec le sérum contenant l'antigène à doser (IgE totales, ou réagines, ou IgG) qui se combine à l'anticorps. Après lavage, le support est mis au contact du même anticorps que le précédent, mais qui a été marqué par un élément radioactif ou un enzyme qui sert de révélateur, comme dans la première méthode.

Dans l'une comme dans l'autre des deux méthodes ci-dessus, on utilise donc un support insoluble qui a fixé soit l'antigène, soit l'anticorps, et cette liaison doit être telle qu'elle ne soit pas rompue par lavage, et que l'antigène ou l'anticorps conserve sa capacité de se combiner réciproquement avec l'anticorps ou l'antigène correspondant.

L'invention, objet de la demande de brevet, concerne un nouveau réactif obtenu pour les dosages radio- et enzymo-immunologiques des immunoglobulines E (IgE), ledit réactif étant constitué d'un support de polysaccharides sur lequel est fixé un allergène. Le réactif selon l'invention convient pour le dosage, dans le sérum des malades allergiques, des immunoglobulines dites "réagines" spécifiques de l'allergène.

Selon les techniques connues, on utilise fréquemment comme supports solides, insolubles, des disques, bandes ou plaques constitués par un polysaccharide, ou un dérivé de polysaccharide, tel que cellulose, dextrane, etc. L'antigène ou l'anticorps, qui est en général une protéine et présente par conséquent des groupes amino est, selon un procédé connu (Brevet français de Pharmacia n° 1.584.535) lié au support par formation de liaison de covalence entre les groupes amino de la protéine et les groupes hydroxyle du support au préalable "activés" par réaction avec un halogénure de cyanogène.

Le réactif pour le dosage immunologique des IgE selon l'invention est obtenu par fixation d'un allergène sur un support insoluble de polysaccharides en mettant en oeuvre une étape d'oxydation des chaînes de polysaccharides par le métaperiodate de sodium et de stabilisation du polysaccharide ainsi activé par le borohydrure de sodium.

Le réactif selon l'invention comprend donc un support polysaccharidique sur lequel on a fixé l'allergène contre lequel sont dirigées les immunoglobulines IgE à doser, par le procédé consistant à:

- dans une première étape, mettre en contact le support avec une solution de métaperiodate de sodium 0,2 M, avec agitation à la température ambiante jusqu'à apparition de fonctions aldéhyde contrôlées sur un prélèvement par le réactif de Schiff;
- dans une seconde étape, coupler le support après rinçage avec un tampon carbonate 0,1 M à pH 9, avec l'allergène dilué dans le même tampon, par incubation à la température ambiante pendant 8 à 15 heures;
- dans une troisième étape, à ajouter à la solution contenant le support, du borohydrure de sodium en poudre jusqu'à une concentration finale de 0,2 M; à laisser incuber 2 heures à la température ambiante, puis à rincer le support d'abord avec le tampon carbonate, puis avec le tampon qui servira au dosage immunologique et à le sécher.

Le procédé d'obtention du réactif selon l'invention est basé sur la réaction connue en elle-même, d'oxydation des chaînes de polysaccharides par le métaperiodate de sodium de formule $NaIO_4$ qui provoque une ouverture des cycles béta-D-glucopyranosyle avec formation de groupes aldéhyde.

Ainsi, lorsqu'on fait incuber la cellulose, formée de chaînes de ribose branchées, avec le catalyseur métaperiodate de sodium, la réaction partielle qui se produit peut être représentée de la façon suivante:

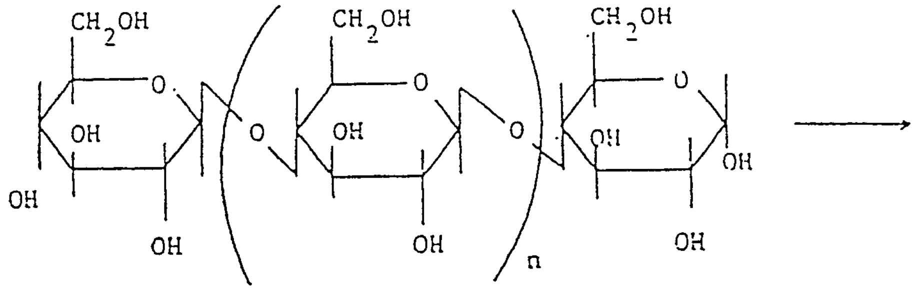

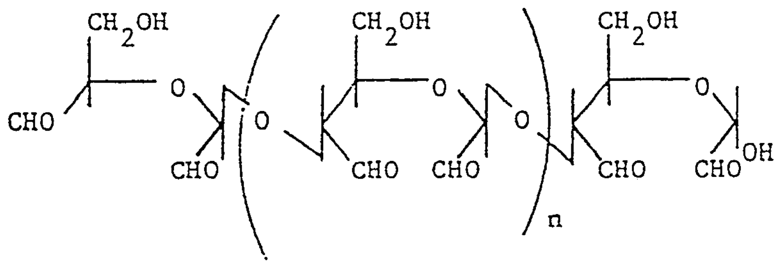

Le polysaccharide ainsi "activé" au métaperiodate peut ensuite être couplé avec une amine de formule $RNH_2$, avec formation d'une liaison imine instable, de formule:

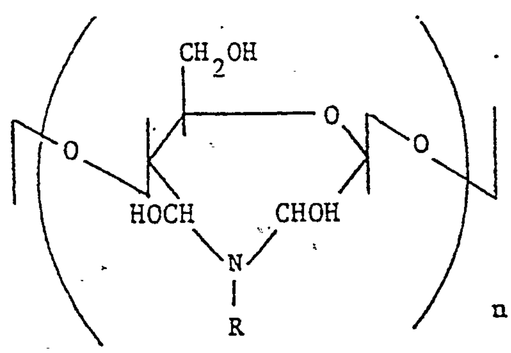

Une réduction par le borohydrure de sodium stabilise le polymère en transformant la liaison imine en liaison amine de formule:

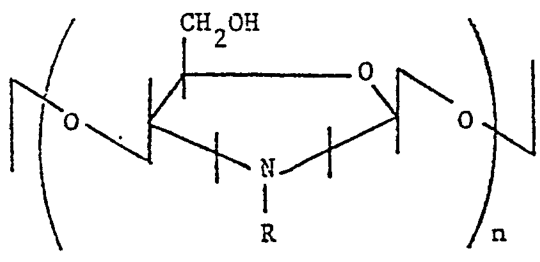

L'amine de formule $RNH_2$ peut être en particulier une protéine ou un peptide, et la série des réactions ci-dessus a été déjà utilisée pour coupler:

- des polysaccharides de pneumocoques aux globules rouges (Rebers et col.: J. Bactériol. 83, 335, 1952);
- des protéines aux globules rouges;
- le dextrane à un anticorps anti-globules rouges (Sanderson et col., Immuno chemistry 8, 163-8, 1971);
- un polysaccharide tel que la cellulose à une molécule porteuse de groupes $NH_2$: le lysoganglioside $GM_1$ (Brevet FR 77/28163 publié sous le n° 2.403.098
- la cellubiose de la fraction d'antigène D à une protéine (brevet FR 77/30521 publié sous le n° 2.382.458).

La réaction d'activation d'un polysaccharide par le métaperiodate, suivie du couplage avec une protéine et de la stabilisation par réduction de la liaison imine formée, a été appliquée, selon l'invention, à la fixation d'un allergène sur un support polysaccharidique, celui-ci pouvant ensuite être utilisé pour la détermination immunologique des immunoglobulines (IgE) (réagines spécifiques d'un allergène).

On a ainsi préparé, selon l'invention des disques de cellulose sur lesquels étaient fixés divers allergènes tels que poussières et moisissures domestiques, pollen de dactyle, graminées, poils de chat, et on a utilisé ces disques avec succès pour la détermination dans le sérum des réagines dirigées spécifiquement contre ces allergènes, soit par une méthode radio-immunologique (RASI) soit par une méthode enzymo-immunologique (ELISA).

Le mode opératoire pour préparer les immunoadsorbants selon l'invention est le suivant:

**a) Activation**

Le support cellulosique (disques de papier par exemple) est mis en contact à température ambiante, sous agitation, pendant 2 heures environ avec une solution 0,2 M de $NaIO_4$ à raison de 100 ml de solution pour 5 à 10 g de cellulose. On rince ensuite le support avec un tampon carbonate 0,1 M - pH 9.

L'apparition des fonctions aldéhyde sur le support cellulosique peut alors être contrôlée par le réactif de Schiff sur un prélèvement (apparition d'une coloration violette).

**b) Couplage de l'allergène**

L'allergène lyophilisé est repris par le tampon carbonate 0,1 M - pH 9 à la concentration voulue.

La cellulose activée est placée dans un tube fermant hermétiquement et on ajoute l'allergène. On fait incuber le tout, sous rotation continue de 8 à 15 heures, de +4°C à la température ambiante.

**c) Stabilisation par réduction**

Du borohydrure de sodium ($NaBH_4$) en poudre est introduit directement dans les tubes de l'opération précédente, de façon à avoir une concentration finale 0,2 M en $NaBH_4$ dans la solution; cette opération est effectuée sous une hotte aspirante à cause du dégagement d'hydrogène. On laisse en contact 2 heures à la température ambiante, puis on rince le support une première fois au tampon carbonate 0,1 M; une deuxième fois au NaCl IM, et une troisième fois avec le tampon phosphate (PBS) qui servira ensuite pour les dosages.

Puis on sèche le support sur papier absorbant et le stocke au congélateur à -25°C.

On a montré que les supports sur lesquels un allergène a été fixé par le procédé selon l'invention, peuvent être utilisés avantageusement pour le dosage des réagines sériques, spécifiques de l'allergène, en les comparant à des supports sur lesquels le même allergène avait été fixé par le procédé connu d'activation préalable au bromure de cyanogène (BrCN).

Dans ce but, on a utilisé à titre d'exemple, deux allergènes lyophilisés:

- Pollen de dactyle (ci-dessous appelé "dactyle") titrant 167.000 unités d'azote protéique (PNU)/ml;
- Poussières et moisissures domestiques (ci-dessous désigné PMD) titrant 179.000 PNU/ml.

Les disques de cellulose sur lesquels on avait fixé l'un de ces allergènes ont été ensuite essayés pour le dosage des réagines spécifiques, par le procédé radio immunologique classique (RASI) en utilisant un pool de sérums positifs (dilué au 1/5) et 3 témoins négatifs:

- témoin HSA (albumine sérique humaine) 0,1 %;
- témoin sérum négatif (dilué au 1/5);
- témoin réactif (sans sérum et avec le réactif anti-IgE pour tenir compte des réactions non-spécifiques).

Les tests RAST étaient réalisés de façon classique. Les disques à étudier sont placés dans les puits de plaques de microtitration et recouverts avec 50 µl de sérum dilue (ou de témoin). On laisse incuber 3 heures à température ambiante sous agitation horizontale continue, puis lave les disques trois fois avec du PBS-tween 20 (4%). On élimine les solutions de lavage et ajoute sur chaque disque 25 µl de reactif anti-IgE marqué à l'iode 125. On couvre la plaque et laisse incuber toute une nuit à température ambiante. Les disques sont ensuite lavés 4 fois avec le tampon PBS-tween et les solutions de lavage sont éliminées. On met les disques secs dans des tubes préalablement repérés et lit au compteur gamma la radio-activité en coups par minute (c.p.m.). Recherche de la concentration optimale d'allergènes à fixer sur les disques de cellulose pour obtenir la plus grande sensibilité dans le dosage des réagines:

Deux lots de disques, l'un activé au BrCN selon la méthode connue, et l'autre au métaperiodate selon le procédé de l'invention, ont été couplés dans les mêmes conditions avec des concentrations croissantes d'allergène: soit 0,5 - 5 - 10 - 15 - 20 - 25 et 30 mg de lyophilisat par ml de diluant; ce qui correspond respectivement pour l'allergène PMD (179.000 PNU/ml) à 60 - 640-1290 - 2000 - 2600 - 3230 et 3800 µg de protéine théorique par ml de diluant, et pour l'allergène dactyle (167.000 PNU/ml) à

78 µg - 783 µg - 1,56 - 2,35 - 3,13 - 3,9 et 4,70 mg par ml de diluant.

Après couplage, les sites réactifs libres sur les disques activés au BrCN sont saturés avec un mélange d'acides aminés ($EPA_{33}$) et les disques activés au métaperiodate sont réduits par le $NaBH_4$ selon le procédé de l'invention.

On effectue le même jour, à l'aide de ces deux lots de disques, des dosages RAST avec le même sérum et le même réactif anti-IgE.

Le tableau I ci-après présente les résultats enregistrés avec les disques activés au bromure de cyanogène, couplés à l'allergène PMD:

Ia) contrôlés avec le sérum positif;

Ib) contrôlés avec le sérum négatif ou HSA. Le témoin réactif est un témoin "bruit de fond".

Le tableau II ci-après présente les résultats enregistrés sur les disques activés avec le métaperiodate, dans les mêmes conditions.

Les tableau, III et IV ci-après présentent les résultats obtenus avec l'allergène dactyle:

III: disques activés au BrCN;

IV: disques activés au métaperiodate.

L'examen de ces tableaux montre:

Pour les disques activés au métaperiodate (tableaux II et IV) selon l'invention, la sensibilité du dosage croit en fonction de la concentration de l'allergène et se stabilise plus ou moins pour une concentration de 15 mg d'allergène/ml (pour atteindre environ 5500 cpm pour le PMD et 7000 cpm pour le dactyle).

Les témoins réactifs ou négatifs évoluent lentement en fonction de la concentration de l'allergène. Les valeurs du témoin réactif ne dépassent pas celles du témoin négatif et varient entre 270 et 340 cpm.

Pour les disques activés au BrCN (tableaux I et III), la sensibilité du dosage croit en fonction de la concentration d'allergène et atteint un plateau à 10 mg d'allergène/ml. La sensibilité des témoins réactifs est indépendante de la concentration de l'allergène, mais est plus élevée que pour les mêmes témoins avec les disques au métaperiodate.

<u>Vérification de la reproductibilité du couplage de l'allergène sur les disques activés.</u>

Après avoir déterminé comme indiqué ci-dessus la concentration optimale de l'allergène à coupler sur les disques préalablement activés, on a incubé des disques préalablement activés, avec la même concentration du même allergène; c'est-à-dire:

10 disques activés au métaperiodate et
10 disques activés au BrCN } couplés avec 10 mg/ml de dactyle;

10 disques activés au métaperiodate et
10 disques activés au BrCN } couplés avec 15 mg/ml de PMD

Les dosages RAST ont été effectués avec les mémes pools de sérums positifs que ceux utilisés précédemment, et avec 6 essais positifs et 4 négatifs par série, et ont été répétés 4 fois. Les résultats sont consignés dans le tableau V ci-après. Ils montrent que la sensibilité des disques activés au métaperiodate est légèrement supérieure à celle des disques activés au BrCN, mais les variabilités au sein de chaque essai sont identiques pour les deux méthodes de fixation et la reproductibilité est bonne d'un essai à l'autre. Les témoins négatifs et réactifs sont bas et très réguliers et le "bruit de fond" (témoin réactif) est légèrement plus faible pour les disques activés selon l'invention. <u>Vérification de la reproductibilité de l'activation des disques par le métaperiodate</u>. 4 lots de disques ont été activés indépendemment les uns des autres avec le métaperiodate. Les disques activés au BRCN faisaient tous partie du même lot d'activation.

Le couplage s'est fait à partir d'une proportion unique d'allergène dactyle (10 mg/ml) et d'allergène PMD (15 mg/ml), répartie sur chaque série de disques.

Les résultats sont consignés dans le tableau VI ci-après. Ils montrent que la sensibilité des disques au métaperiodate n'est pas modifiée d'un lot d'activation à l'autre. La variabilité de la sensibilité au sein d'un lot recoupe assez bien celle enregistrée avec les disques activés au BrCN.

Mais pour les disques activés au métaperiodate les témoins negatifs sont bas et peu variables et les témoins réactifs témoignent d'un bruit de fond faible.

En conclusion, la sensibilité des dosages RAST avec lés disques activés au métaperiodate selon l'invention est aussi bonne et même légèrement supérieure à celle des mêmes dosages effectués avec les disques activés au BrCN.

La reproductibilité de l'activation par le métaperiodate et le couplage, selon l'invention, avec les allergènes étudiés est tout à fait satisfaisante.

Les bruits de fond donnés par le témoin réactif et les témoins négatifs sont plus faibles avec les disques activés selon l'invention qu'avec ceux activés par le BrCN, d'où une sensibilité plus grande pour les sérums faiblement positifs.

Les expériences précédentes ont montré que les disques activés au métaperiodate conviennent au, dosages RAST pour un certain nombre d'allergènes. D'autres expériences ont permis de comparer les disques activés au métaperiodate et les disques activés au bromure de cyanogène, lorsqu'on fixe sur ces disques des extraits d'allergènes très divers:

- Poussière de maison, Dermatophagoides pteronyssinus, poil de chat, cinq graminées, bouleau.

Les disques de papier activés au bromure de cyanogène d'une part, et au métaperiodate d'autre part, ont été couplés avec les allergènes ci-dessus (approximativement 1 ml par disque), comme décrit préalablement.

La sensibilité des disques activés au bromure de cyanogène d'une part, et activés au métaperiodate de l'autre, et couplés aux allergènes ci-dessus, a été étudiée directement par la méthode RAST. On incube 50 µl de sérum humain à la dilution 1,5 dans une solution saline tamponnée au phosphate (s.t.p.) avec un disque couplé

avec un allergène pendant trois heures à la température ambiante. On lave ensuite le disque soigneusement en utilisant du s.t.p. contenant 1 % de Tween 20, puis on mesure l'IgE combiné aux disques en utilisant de l'anti-IgE marqué par $^{125}$ I.

La quantité d'IgE combinée aux disques activés au bromure de cyanogène et couplés à l'allergène fut comparée à la quantité d'IgE combinée aux disques activés au métaperiodate et couplés à l'allergène.

Les résultats sont donnés dans les tableaux VII à XI ci-après.

En conclusion, la sensibilité des disques activés au métapcriodate, mesurée en coupspar minute (anti-IgE marqué par $^{125}$I correspondant à l'IgE combiné aux disques) s'est révélée au moins équivalente, et dans plusieurs cas, supérieure à celle des disques de papier activés au bromure de cyanogène, quand on les utilise pour un grand nombre d'allergènes.

D'autre part, une série d'essais ont été faits pour utiliser les disques activés et couplés selon L'invention avec divers allergènes, dans les procédés de dosage enzymo-immunologiques (ELISA) des réagines spécifiques des allergènes: Les disques sur lesquels un allergène a été fixé selon l'invention sont mis en contact avec un pool de sérum contenant les IgE à doser, spécifiques de l'allergène. Ces IgE se combinent sélectivement à un anti-IgE spécifique du fragment Fc de l'allergène, couplé à un enzyme que l'on peut déceler lors de la dégradation de son substrat. Le disque est alors éliminé, et le substrat coloré, en phase liquide, est lu au colorimètre.

On a ainsi pû observer la même sensibilité et reproductibilité des dosages ELISA réalisés avec les disques selon l'invention, que lors des dosages RAST effectués avec les mêmes disques.

Les supports cellulosiques activés et couplés selon l'invention avec un allergène peuvent donc être utilises avec succès aussi bien pour les dosages radio-immunologiques que pour les dosages enzymo-immunologiques des IgE, tout en presentant par rapport aux supports activés par la méthode connue au bromure de cyanogène, l'avantage de n'être pas toxique.

TABLEAU I.a
SENSIBILITE DES DISQUES BrCN-PMD MESUREE PAR LE RAST

**Sérum positif**

| Activité (c.p.m.) / Concentration d'allergène en mg de lyophilisat/ml | 0,5 | 5 | 10 | 15 | 20 | 25 | 30 |
|---|---|---|---|---|---|---|---|
| Moyenne sur 6 disques | 3853 | 5116 | 5381 | 5139 | 5531 | 5564 | 5180 |
| Variation standard | ± 159 | ± 196 | ± 304 | ± 301 | ± 316 | ± 506 | ± 161 |
| Pourcentage de variation | 4 % | 4 % | 6 % | 6 % | 6 % | 9 % | 3 % |

TABLEAU I.b
SENSIBILITE DES DISQUES BrCN-PMD MESUREE PAR LE RAST

Témoins négatifs

| | Activité (c.p.m.) / Concentration d'allergène en mg de lyophilisat/ml | 0,5 | 5 | 10 | 15 | 20 | 25 | 30 |
|---|---|---|---|---|---|---|---|---|
| Témoin | Moyenne sur 2 disques | 303 | 300 | 365 | 346 | 348 | 325 | 309 |
| HSA | Variation standard | ± 18 | ± 25 | ± 55 | ± 51 | ± 37 | ± 29 | ± 29 |
| 0,1 % | Pourcentage de variation | 6 % | 8 % | 15 % | 15 % | 11 % | 9 % | 9 % |
| Témoin | Moyenne sur 2 disques | 277 | 303 | 287 | 296 | 309 | 264 | 262 |
| Sérum | Variation standard | ± 72 | ± 78 | ± 44 | ± 8 | ± 13 | ± 20 | ± 3 |
| négatif | Pourcentage de variation | 26 % | 26 % | 15 % | 3 % | 4 % | 8 % | 1 % |
| Témoin réactif | Moyenne sur 2 disques | 383 | 400 | 331 | 364 | 402 | 418 | 371 |
| | Variation standard | ± 75 | ± 59 | ± 7 | ± 37 | ± 54 | ± 0 | ± 13 |
| | Pourcentage de variation | 20 % | 15 % | 2 % | 10 % | 13 % | 0 % | 4 % |

TABLEAU II.a
SENSIBILITE DES DISQUES METAPERIODATE-PMD MESUREE PAR LE RAST

Sérum positif

| Activité (c.p.m.) / Concentration d'allergène en mg de lyophilisat/ml | 0,5 | 5 | 10 | 15 | 20 | 25 | 30 |
|---|---|---|---|---|---|---|---|
| Moyenne sur 6 disques | 4785 | 5990 | 5890 | 5714 | 6013 | 5720 | 6041 |
| Variation standard | ± 862 | ± 467 | ± 346 | ± 228 | ± 335 | ± 373 | ± 144 |
| Pourcentage de variation | 18 % | 8 % | 6 % | 4 % | 6 % | 7 % | 2 % |

TABLEAU II.b
SENSIBILITE DES DISQUES METAPERIODATE-PMD MESUREE PAR LE RAST

Témoins négatifs

| | Activité (c.p.m.) / Concentration d'allergène en mg de lyophilisat/ml | 0,5 | 5 | 10 | 15 | 20 | 25 | 30 |
|---|---|---|---|---|---|---|---|---|
| Témoin | Moyenne sur 2 disques | NF | 161 | 203 | 156 | 189 | 183 | 224 |
| HSA | Variation standard | NF | ± 41 | ± 13 | ± 25 | ± 24 | ± 30 | ± 37 |
| 0,1 % | Pourcentage de variation | NF | 25 % | 6 % | 16 % | 13 % | 16 % | 17 % |
| Témoin | Moyenne sur 2 disques | 196 | 231 | 223 | 225 | 237 | 226 | 201 |
| Sérum | Variation standard | NF | ± 15 | ± 27 | ± 24 | ± 106 | ± 14 | ± 13 |
| négatif | Pourcentage de variation | NF | 6 % | 12 % | 11 % | 45 % | 6 % | 6 % |
| Témoin réactif | Moyenne sur 2 disques | 158 | 165 | 248 | 191 | 206 | 203 | 182 |
| | Variation standard | NF | ± 10 | ± 34 | ± 35 | ± 23 | ± 1 | ± 31 |
| | Pourcentage de variation | NF | 6 % | 14 % | 18 % | 11 % | 0 % | 17 % |

TABLEAU III.a
SENSIBILITE DES DISQUES BrCN-DACTYLE MESUREE PAR LE RAST

Sérum positif

| Activité (c.p.m.) / Concentration d'allergène en mg de lyophilisat/ml | 0,5 | 5 | 10 | 15 | 20 | 25 | 30 |
|---|---|---|---|---|---|---|---|
| Moyenne sur 6 disques | 6312 | 7175 | 7346 | 7466 | 7552 | 7523 | 7561 |
| Variation standard | ± 306 | ± 464 | ± 212 | ± 211 | ± 269 | ± 269 | ± 306 |
| Pourcentage de variation | 5 % | 6 % | 3 % | 3 % | 4 % | 4 % | 4 % |

TABLEAU III.b
SENSIBILITE DES DISQUES BrCN-DACTYLE MESUREE PAR LE RAST
Témoins négatifs

| | Activité (c.p.m.) / Concentration d'allergène en mg de lyophilisat/ml | 0,5 | 5 | 10 | 15 | 20 | 25 | 30 |
|---|---|---|---|---|---|---|---|---|
| Témoin | Moyenne sur 2 disques | 224 | 276 | 469 | 420 | 435 | 502 | 383 |
| HSA | Variation standard | ± 195 | ± 263 | ± 10 | ± 45 | ± 78 | ± 136 | ± 55 |
| 0,1 % | Pourcentage de variation | 87 % | 95 % | 2 % | 11 % | 18 % | 27 % | 14 % |
| Témoin | Moyenne sur 2 disques | 361 | 319 | 422 | 366 | 384 | 378 | 515 |
| Sérum | Variation standard | ± 61 | ± 27 | ± 14 | ± 0 | ± 3 | ± 31 | ± 69 |
| négatif | Pourcentage de variation | 17 % | 8 % | 3 % | 0 % | 1 % | 8 % | 13 % |
| Témoin réactif | Moyenne sur 2 disques | 451 | 496 | 443 | 540 | 496 | 497 | 445 |
| | Variation standard | ± 1 | ± 110 | ± 66 | ± 25 | ± 39 | ± 33 | ± 38 |
| | Pourcentage de variation | 0 % | 22 % | 15 % | 5 % | 8 % | 7 % | 9 % |

TABLEAU IV.a
SENSIBILITE DES DISQUES METAPERIODATE-DACTYLE MESUREE PAR LE RAST
Sérum positif

| Activité (c.p.m.) / Concentration d'allergène en mg de lyophilisat/ml | 0,5 | 5 | 10 | 15 | 20 | 25 | 30 |
|---|---|---|---|---|---|---|---|
| Moyenne sur 6 disques | 5433 | 6642 | 6847 | 6937 | 7608 | 6895 | 7170 |
| Variation standard | ± 482 | ± 439 | ± 291 | ± 178 | ± 246 | ± 402 | ± 325 |
| Pourcentage de variation | 9 % | 7 % | 4 % | 3 % | 3 % | 6 % | 5 % |

TABLEAU IV.b
SENSIBILITE DES DISQUES METAPERIODATE-DACTYLE MESUREE PAR LE RAST

Témoins négatifs

| | Activité (c.p.m.) / Concentration d'allergène en mg de lyophilisat/ml | 0,5 | 5 | 10 | 15 | 20 | 25 | 30 |
|---|---|---|---|---|---|---|---|---|
| Témoin | Moyenne sur 2 disques | 242 | 297 | 325 | 376 | 419 | 391 | 398 |
| HSA | Variation standard | ± 20 | ± 27 | ± 32,5 | ± 8 | ± 29 | ± 24 | ± 34 |
| 0,1 % | Pourcentage de variation | 8 % | 9 % | 10 % | 2 % | 7 % | 6 % | 9 % |
| Témoin | Moyenne sur 2 disques | 205 | 291 | 344 | 404 | 379 | 407 | 481 |
| Sérum | Variation standard | ± 10 | ± 15 | ± 20 | ± 17 | ± 81 | ± 24 | ± 27 |
| négatif | Pourcentage de variation | 5 % | 5 % | 6 % | 4 % | 21 % | 6 % | 6 % |
| Témoin réactif | Moyenne sur 2 disques | 242 | 314 | 357 | 361 | 372 | 401 | 406 |
| | Variation standard | ± 20 | ± 20 | ± 38 | ± 35 | ± 79 | ± 4 | ± 8 |
| | Pourcentage de variation | 8 % | 6 % | 11 % | 10 % | 21 % | 1 % | 2 % |

TABLEAU V — REPRODUCTIBILITE DU COUPLAGE

Activité en c.p.m. (RAST)

| Allergène / N° de couplage | Contrôle | avec disques BrCN | | avec disques métaperiodate | |
|---|---|---|---|---|---|
| | | PMD | DACTYLE | PMD | DACTYLE |
| 1 | Sérum + | 4650 ± 268 | 6571 ± 234 | 5539 ± 219 | 6307 ± 298 |
| | Sérum — | 194 ± 39 | 281 ± 35 | 225 ± 114 | 188 ± 03 |
| | Réactif | 288 ± 03 | 330 ± 03 | 154 ± 06 | 193 ± 24 |
| 2 | Sérum + | 4407 ± 657 | 5511 ± 637 | 5589 ± 381 | 6220 ± 348 |
| | Sérum — | 200 ± 59 | 272 ± 79 | 143 ± 21 | 182 ± 09 |
| | Réactif | 247 ± 61 | 297 ± 44 | 177 ± 4 | 196 ± 06 |
| 3 | Sérum + | 4428 ± 677 | 5412 ± 242 | 5478 ± 273 | 6298 ± 319 |
| | Sérum — | 241 ± 27 | 254 ± 08 | 181 ± 15 | 219 ± 04 |
| | Réactif | 314 | 343 ± 47 | 168 ± 28 | 196 ± 62 |
| 4 | Sérum + | 5024 ± 861 | 5286 ± 314 | 5304 ± 463 | 5954 ± 390 |
| | Sérum — | 232 ± 14 | 228 ± 08 | 195 ± 13 | 201 ± 55 |
| | Réactif | 310 ± 23 | 337 ± 15 | 141 ± 01 | 207 ± 44 |

TABLEAU VI — REPRODUCTIBILITE D'ACTIVATION

Activité en c.p.m. (RAST)

| Allergène / N° de couplage | Contrôle | avec disques BrCN PMD | avec disques BrCN DACTYLE | avec disques métaperiodate PMD | avec disques métaperiodate DACTYLE |
|---|---|---|---|---|---|
| 1 | Sérum + | 4382 ± 357 | 5679 ± 208 | 4351 ± 269 | 5125 ± 365 |
| | Sérum — | 318 ± 5 | 366 ± 136 | 188 ± 17 | 266 ± 28 |
| | Réactif | 402 ± 31 | 425 ± 27 | 171 ± 07 | 269 ± 15 |
| 2 | Sérum + | 4467 ± 188 | 5628 ± 385 | 5066 ± 236 | 5210 ± 215 |
| | Sérum — | 286 ± 8 | 384 ± 28 | 168 | 233 ± 4 |
| | Réactif | 351 ± 30 | 488 ± 03 | 180 | 265 ± 24 |
| 3 | Sérum + | 4138 ± 364 | 5292 ± 638 | 4630 ± 413 | 5326 ± 73 |
| | Sérum — | 311 ± 58 | 373 ± 117 | 171 ± 21 | 244 ± 22 |
| | Réactif | 497 ± 64 | 442 ± 10 | 196 ± 48 | 180 ± 31 |
| 4 | Sérum + | 4168 ± 233 | 5376 ± 359 | 4490 ± 274 | 5346 ± 115 |
| | Sérum — | 259 ± 7 | 396 ± 11 | 180 ± 45 | 214 ± 23 |
| | Réactif | 369 ± 44 | 529 ± 38 | 196 ± 23 | 181 ± 41 |

**TABLEAU VII — Poussière de maison**
**Coups par minute** (IgE combiné)

| Sérum | Disques activés au bromure de cyanogène | Disques activés au metaperiodate |
|---|---|---|
| 1 | 274 | 350 |
| 2 | 2880 | 3152 |
| 3 | 248 | 450 |
| 4 | 736 | 3122 |
| 5 | 190 | 368 |
| 6 | 1728 | 3822 |
| 7 | 198 | 446 |
| 8 | 272 | 438 |
| 9 | 1112 | 2420 |
| 10 | 236 | 434 |

**TABLEAU VIII — Dermatophagoides ptéronyssinus**
**Coups par minute** (IgE combiné)

| Sérum | Disques activés au bromure de cyanogène | Disques activés au metaperiodate |
|---|---|---|
| 1 | 210 | 348 |
| 2 | 2836 | 9892 |
| 3 | 186 | 220 |
| 4 | 222 | 222 |
| 5 | 378 | 1694 |
| 6 | 226 | 298 |
| 7 | 1046 | 8620 |
| 8 | 194 | 246 |
| 9 | 208 | 206 |
| 10 | 238 | 346 |

TABLEAU IX — Poil de chat
Coups par minute (IgE combiné)

| Sérum | Disques activés au bromure de cyanogène | Disques activés au metaperiodate |
|---|---|---|
| 1 | 356 | 496 |
| 2 | 176 | 392 |
| 3 | 6545 | 8966 |
| 4 | 8430 | 10121 |
| 5 | 250 | 346 |

TABLEAU X — Cinq graminées
Coups par minute (IgE combiné)

| Sérum | Disques activés au bromure de cyanogène | Disques activés au metaperiodate |
|---|---|---|
| 1 | 250 | 370 |
| 2 | 360 | 1264 |
| 3 | 338 | 732 |
| 4 | 4748 | 11742 |
| 5 | 310 | 460 |

TABLEAU XI — Bouleau
Coups par minute (IgE combiné)

| Sérum | Disques activés au bromure de cyanogène | Disques activés au metaperiodate |
|---|---|---|
| 1 | 4368 | 5976 |
| 2 | 4671 | 6660 |
| 3 | 4553 | 5930 |
| 4 | 754 | 2280 |

**Revendications**

1. Réactif pour le dosage radio- ou enzymoimmunologique des IgE, caractérisé en ce qu'il comprend un support polysaccharidique sur lequel on a fixé l'allergène contre lequel sont dirigées les immunoglobulines IgE à doser, par le procédé consistant à:
- dans une première étape, mettre en contact le support avec une solution de métaperiodate de sodium 0,2 M, avec agitation à la température ambiante jusqu'à apparition de fonctions aldéhyde contrôlées sur un prélèvement par le réactif de Schiff;
- dans une seconde étape, coupler le support après rinçage avec un tampon carbonate 0,1 M à pH 9, avec l'allergène dilué dans le meme tampon, par incubation à la température ambiante pendant 8 à 15 heures;
- dans une troisième étape, à ajouter à la solution contenant le support, du borohydrure de sodium en poudre jusqu'à une concentration finale de 0,2 M; à laisser incuber 2 heures à la température ambiante, puis à rincer le support d'abord avec le tampon carbonate, puis avec le tampon qui servira au dosage immunologique et à le sécher.

2. Réactif selon la revendication 1, caractérisé en ce que le support est constitué par de la cellulose ou des dérivés de la cellulose et en particulier des disques de papier.

3. Réactif selon l'une des revendications 1 ou 2, caractérisé en ce que l'allergène fixé sur le support cst un mélangede poussières et de moisissures domestiques, un pollen de dactyle, un mélange de graminées, des poils de chat ou Dermatophagoides ptéronyssinus.

4. Réactif selon l'une quelconque des revendications 1 à 3, caractérisé en ce que la concentration optimale de l'allergène à coupler sur le support est de 10 à 15 mg d'allergène lyophilisé par ml de diluants.

**Claims**

1. A reagent for radio or enzyme-immunological IgE analysis, characterised in that it comprises a polysaccharide support on which the allergen against which the IgE immunoglobulins for analysis are directed has been fixed by the method comprising:
- in a first step, bringing the support into contact with an 0.2 M solution of sodium metaperiodate, with agitation at ambient temperature until aldehyde groups appear as checked on a sample by means of Schiff's reagent,

- in a second step, coupling the support, after rinsing with an 0.1 M carbonate buffer at pH 9, with the allergen diluted in the same buffer, by incubation at ambient temperature for 8 to 15 hours,

- in a third step, adding powdered sodium borohydride to the support-containing solution to a final concentration of 0.2 M, incubating for 2 hours at ambient temperature, then rinsing the support first with the carbonate buffer and then with the buffer which will be used for the immunological analysis and drying it.

2. A reagent according to claim 1, characterised in that the support consists of cellulose or cellulose derivatives and particularly of paper discs.

3. A reagent according to claim 1 or 2, characterised in that the allergen fixed on the support is a mixture of domestic moulds and dusts, a dactylis pollen, a mixture of Graminaceae, cat's hairs or Dermatophagoides pteronyssinus.

4. A reagent according to any one of claims 1 to 3, characterised in that the optimum concentration of the allergen to be coupled on the support is 10 - 15 mg of lyophilized allergen per ml of diluents.

## Patentansprüche

1. Reagenz zur radio- oder enzymimmunologischen Bestimmung der IgE, dadurch gekennzeichnet, daß es einen Polysaccharid-Träger aufweist, an den das Allergen, gegen das die zu bestimmenden Immunoglobuline gerichtet sind, durch das folgende Schritte umfassende Verfahren gebunden ist:

- in einem ersten Schritt den Träger mit einer 0,2 m Natriummetajodat-Lösung unter Rühren bei Raumtemperatur in Berührung bringen bis zum Auftreten von Aldehyd-Gruppen, die durch eine Stichprobe mit Schiffs Reagenz kontrolliert werden;

- in einem zweiten Schritt den Träger nach dem Spülen mit einem 0,1 m Karbonatpuffer von pH 9 mit dem in demselben Puffer verdünnten Allergen durch 8 bis 15 Stunden dauernde Inkubation bei Raumtemperatur verbinden;

- in einem dritten Schritt der den Träger enthaltenden Lösung pulverförmiges Natriumborhydrid bis zu einer 0,2 m Endkonzentration zufügen, bei Raumtemperatur 2 Stunden inkubieren lassen, dann den Träger zunächst mit dem Karbonatpuffer spülen, dann mit dem Puffer, der zu der immunologischen Bestimmung dienen soll, und dann trocknen.

2. Reagenz nach Anspruch 1, dadurch gekennzeichnet, daß der Träger aus Zellulose oder Zellulosederivaten, insbesondere aus Papierscheiben besteht.

3. Reagenz nach Anspruch 1 oder 2, dadurch gekennzeichnet, daß das an den Träger gebundene Allergen ein Gemisch von Hausstaub und Hausschimmel ist, ein Knäuelgraspollen, ein Gramineengemisch, Katzenhaar oder Hausstaubmilben (Dermatophagoides pteronyssinus).

4. Reagenz nach einem der Ansprüche 1 bis 3, dadurch gekennzeichnet, daß die optimale Konzentration des an den Träger zu bindenden Allergens 10 bis 15 mg gefriergetrocknetes Allergen je Milliliter Verdünnungsmittel beträgt.